# EUROPÄISCHE PATENTANMELDUNG

(11) **EP 4 369 353 A1**
(43) Veröffentlichungstag der Anmeldung: **15.05.2024**
(21) Anmeldenummer: 22207079.9
(22) Anmeldetag: 12.11.2022
(51) Int. Cl.: G16H 30/20

(54) **ERZEUGUNG VON KÜNSTLICHEN KONTRASTMITTELVERSTÄRKTEN RADIOLOGISCHEN AUFNAHMEN**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die vorliegende Offenbarung befasst sich mit dem technischen Gebiet der Erzeugung von künstlichen kontrastmittelverstärkten radiologischen Aufnahmen.

## Beschreibung

Die vorliegende Offenbarung befasst sich mit dem technischen Gebiet der Erzeugung von künstlichen kontrastmittelverstärkten radiologischen Aufnahmen.

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In dem offenbarten Verfahren wird in einem ersten Schritt ein Trainingsdatensatz erzeugt. Der Trainingsdatensatz umfasst für eine Vielzahl an Personen für jede Person i) eine native radiologische Aufnahme (*zero-contrast image*), ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*).

In einem zweiten Schritt wird ein künstliches neuronales Netz trainiert, für jede Person des Trainingsdatensatzes auf Basis der nativen Aufnahme und der Aufnahme nach Applikation einer geringen Menge an Kontrastmittel eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringen Menge an Kontrastmittel eine künstliche radiologische Aufnahme vorherzusagen, die den aufgenommenen Bereich so zeigt wie er aussehen würde, wenn eine Standardmenge an Kontrastmittel appliziert worden wäre.

Das in WO2019/074938A1 offenbarte Verfahren weist Nachteile auf.

So sind für das Trainieren des künstlichen neuronalen Netzes Trainingsdaten erforderlich. Es müssen eine Vielzahl an radiologischen Untersuchungen an einer Vielzahl an Personen vorgenommen und die Trainingsdaten generiert werden, um das Netz trainieren zu können.

Das in WO2019/074938A1 offenbarte künstliche neuronale Netz ist trainiert, eine radiologische Aufnahme nach der Applikation einer Standardmenge eines Kontrastmittels vorherzusagen. Das künstliche neuronale Netz ist nicht konfiguriert und nicht trainiert, eine radiologische Aufnahme nach der Applikation einer geringeren oder höheren Menge als der Standarmenge an Kontrastmittel vorherzusagen. Das in WO2019/074938A1 beschriebene Verfahren kann prinzipiell trainiert werden, eine radiologische Aufnahme nach der Applikation einer anderen Menge als der Standardmenge an Kontrastmittel vorherzusagen. Allerdings sind hierfür weitere Trainingsdaten und ein weiteres Training erforderlich.

Es wäre wünschenswert, radiologische Aufnahmen mit einer variablen Kontrastverstärkung erzeugen zu können, ohne dass für jede einzelne Kontrastverstärkung Trainingsdaten erzeugt und ein künstliches neuronales Netz trainiert werden müssen. Es wäre weiterhin wünschenswert, radiologische Aufnahmen mit einer variablen Kontrastverstärkung erzeugen zu können, wobei ein nachvollziehbarer, deterministischer Prozess zur Erzeugung der variablen Kontrastverstärkung angewendet wird. Dies erleichtert Zulassung und Anwendung eines entsprechenden Verfahrens im medizinischen Bereich, in dem falsch negative und falsch positive Befunde zu minimieren sind. Methoden des maschinellen Lernens nutzen statistische Modelle, deren Generalisierbarkeit beschränkt ist, da ihnen üblicherweise eine beschränkte Auswahl an Trainingsdaten zu Grunde liegt. Es wäre weiterhin wünschenswert, radiologische Aufnahmen mit einer variablen Kontrastverstärkung unter Verwendung einer breiten Vielfalt von Kontrastmitteln erzeugen zu können. Es wäre weiterhin wünschenswert, das Verfahren zur Erzeugung radiologischer Aufnahmen mit einer variablen Kontrastverstärkung mit einer breiten Vielfalt verschiedener Kontrastmittel unabhängig von ihren physischen, chemischen, physiologischen oder sonstigen Eigenschaften anwenden zu können.

Diese und weitere Aufgaben werden durch die Gegenstände der unabhängigen Patentansprüche gelöst. Bevorzugte Ausführungsformen der vorliegenden Offenbarung finden sich in den abhängigen Patentansprüchen, in der vorliegenden Beschreibung und in den Zeichnungen.

Ein erster Gegenstand der vorliegenden Offenbarung ist somit ein computer-implementiertes Verfahren zur Erzeugung einer kontrastverstärkten radiologischen Aufnahme, umfassend die Schritte:
- Empfangen einer ersten Repräsentation eines Untersuchungsbereichs eines Untersuchungsobjekts, wobei die erste Repräsentation den Untersuchungsbereich ohne Kontrastmittel oder nach Applikation einer ersten Menge eines Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

Ein weiterer Gegenstand der vorliegenden Offenbarung ist ein Computersystem umfassend:
einen Prozessor; und
einen Speicher, der ein Anwendungsprogramm speichert, das so konfiguriert ist, dass es, wenn es vom Prozessor ausgeführt wird, eine Operation durchführt, wobei die Operation umfasst:
   - Empfangen einer ersten Repräsentation eines Untersuchungsbereichs eines Untersuchungsobjekts, wobei die erste Repräsentation den Untersuchungsbereich ohne Kontrastmittel oder nach Applikation einer ersten Menge eines Kontrastmittels repräsentiert,
   - Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
   - Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
   - Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
   - Ausgeben und/oder Speichern der vierten Repräsentation und/oder Übermitteln der vierten Repräsentation an ein separates Computersystem.

Ein weiterer Gegenstand der vorliegenden Offenbarung ist ein Computerprogramm, das in einen Arbeitsspeicher eines Computersystems geladen werden kann und dort das Computersystem dazu veranlasst, folgende Schritte ausführen:
- Empfangen einer ersten Repräsentation eines Untersuchungsbereichs eines Untersuchungsobjekts, wobei die erste Repräsentation den Untersuchungsbereich ohne Kontrastmittel oder nach Applikation einer ersten Menge eines Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

Ein weiterer Gegenstand der vorliegenden Offenbarung ist eine Verwendung eines Kontrastmittels in einer radiologischen Untersuchung umfassend:
- Empfangen oder Erzeugen einer ersten Repräsentation eines Untersuchungsbereichs eines Untersuchungsobjekts, wobei die erste Repräsentation den Untersuchungsbereich ohne das Kontrastmittel oder nach Applikation einer ersten Menge des Kontrastmittels repräsentiert,
- Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

Ein weiterer Gegenstand der vorliegenden Offenbarung ist ein Kontrastmittel zur Verwendung in einem radiologischen Untersuchungsverfahren umfassend:
- Empfangen oder Erzeugen einer ersten Repräsentation eines Untersuchungsbereichs eines Untersuchungsobjekts, wobei die erste Repräsentation den Untersuchungsbereich ohne das Kontrastmittel oder nach Applikation einer ersten Menge des Kontrastmittels repräsentiert,
- Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

Ein weiterer Gegenstand der vorliegenden Offenbarung ist ein Kit umfassend ein Computerprogrammprodukt und ein Kontrastmittel, wobei das Computerprogrammprodukt ein Computerprogramm umfasst, das in einen Arbeitsspeicher eines Computersystems geladen werden kann und dort das Computersystem dazu veranlasst, folgende Schritte ausführen:
- Empfangen einer ersten Repräsentation eines Untersuchungsbereichs eines Untersuchungsobjekts, wobei die erste Repräsentation den Untersuchungsbereich ohne das Kontrastmittel oder nach Applikation einer ersten Menge des Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

Die Gegenstände der vorliegenden Offenbarung werden nachstehend näher erläutert, ohne zwischen den Gegenständen (Verfahren, Computersystem, Computerprogramm(produkt), Verwendung, Kontrastmittel zur Verwendung, Kit) zu unterscheiden. Die nachfolgenden Erläuterungen sollen vielmehr für alle Gegenstände in analoger Weise gelten, unabhängig davon, in welchem Kontext (Verfahren, Computersystem, Computerprogramm(produkt), Verwendung, Kontrastmittel zur Verwendung, Kit) sie erfolgen.

Wenn in der vorliegenden Beschreibung oder in den Patentansprüchen Schritte in einer Reihenfolge genannt sind, bedeutet dies nicht zwingend, dass diese Offenbarung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Die vorliegende Offenbarung beschreibt Mittel, mit denen auf Basis von mindestens zwei Repräsentationen, die einen Untersuchungsbereich eines Untersuchungsobjekts nach Zugabe/Anwendung/Verwendung unterschiedlicher Mengen an Kontrastmittel repräsentieren, eine oder mehrere künstliche radiologische Aufnahmen erzeugt werden, bei der/denen der Kontrast zwischen Bereichen mit Kontrastmittel und Bereichen ohne Kontrastmittel variiert werden kann.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs oder mehrere Organe oder ein anderer Teil des Untersuchungsobjekts.

Der Untersuchungsbereich kann beispielsweise eine Leber, eine Niere, ein Herz, eine Lunge, ein Gehirn, ein Magen, eine Blase, eine Prostatadrüse, ein Darm oder ein Teil davon oder ein anderer Teil des Körpers eines Säugetiers (z.B. eines Menschen) sein.

In einer Ausführungsform umfasst der Untersuchungsbereich eine Leber oder einen Teil einer Leber oder der Untersuchungsbereich ist eine Leber oder ein Teil einer Leber eines Säugetiers, vorzugsweise eines Menschen.

In einer weiteren Ausführungsform umfasst der Untersuchungsbereich ein Gehirn oder einen Teil eines Gehirns oder der Untersuchungsbereich ist ein Gehirn oder ein Teil eines Gehirns eines Säugetiers, vorzugsweise eines Menschen.

In einer weiteren Ausführungsform umfasst der Untersuchungsbereich ein Herz oder ein Teil eines Herzes oder der Untersuchungsbereich ist ein Herz oder ein Teil eines Herzes eines Säugetiers, vorzugsweise eines Menschen.

In einer weiteren Ausführungsform umfasst der Untersuchungsbereich einen Thorax oder einen Teil eines Thorax oder der Untersuchungsbereich ist ein Thorax oder ein Teil eines Thorax eines Säugetiers, vorzugsweise eines Menschen.

In einer weiteren Ausführungsform umfasst der Untersuchungsbereich einen Magen oder einen Teil eines Magens oder der Untersuchungsbereich ist ein Magen oder ein Teil eines Magens eines Säugetiers, vorzugsweise eines Menschen.

In einer weiteren Ausführungsform umfasst der Untersuchungsbereich eine Bauchspeicheldrüse oder einen Teil einer Bauchspeicheldrüse oder der Untersuchungsbereich ist eine Bauchspeicheldrüse oder ein Teil einer Bauchspeicheldrüse eines Säugetiers, vorzugsweise eines Menschen.

In einer weiteren Ausführungsform umfasst der Untersuchungsbereich eine Niere oder einen Teil einer Niere oder der Untersuchungsbereich ist eine Niere oder ein Teil einer Niere eines Säugetiers, vorzugsweise eines Menschen.

In einer weiteren Ausführungsform umfasst der Untersuchungsbereich einen oder beide Lungenflügel oder einen Teil eines Lungenflügels Säugetiers, vorzugsweise eines Menschen.

In einer weiteren Ausführungsform umfasst der Untersuchungsbereich eine Brust oder einen Teil einer Brust oder der Untersuchungsbereich ist eine Brust oder ein Teil einer Brust eines weiblichen Säugetiers, vorzugsweise eines weiblichen Menschen.

In einer weiteren Ausführungsform umfasst der Untersuchungsbereich eine Prostata oder einen Teil einer Prostata oder der Untersuchungsbereich ist eine Prostata oder ein Teil einer Prostata eines männlichen Säugetiers, vorzugsweise eines männlichen Menschen.

Der Untersuchungsbereich, auch Aufnahmevolumen (engl.: *field of view*, FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird einer radiologischen Untersuchung unterzogen.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Offenbarung umfasst damit insbesondere die folgenden Untersuchungsmethoden: Computertomografie, Magnetresonanztomografie, Sonografie.

In einer Ausführungsform der vorliegenden Offenbarung handelt es sich bei der radiologischen Untersuchung um eine magnetresonanztomographische Untersuchung.

In einer weiteren Ausführungsform handelt es sich bei der radiologischen Untersuchung um eine computertomografische Untersuchung

In einer Ausführungsform handelt es sich bei der radiologischen Untersuchung um eine Ultraschalluntersuchung.

Bei radiologischen Untersuchungen werden häufig Kontrastmittel zur Kontrastverstärkung eingesetzt.

"Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate) als Kontrastmittel verwendet. Im Fall der Sonografie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wpcontent/uploads/2017/10/contrast-agents-tutorial.pdf, M. R. Nough et al.: Radiographic and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}), Gadobutrol (Gadovist^{®}) und Gadoxetsäure (Primovist^{®}/Eovist^{®}).

Die Erzeugung einer künstlichen radiologischen Aufnahme mit einer variablen Kontrastverstärkung basiert auf mindestens zwei Repräsentationen des Untersuchungsbereichs, einer ersten Repräsentation und einer zweiten Repräsentation.

Die erste Repräsentation repräsentiert den Untersuchungsbereich ohne Kontrastmittel oder nach der Applikation einer ersten Menge eines Kontrastmittels. Vorzugsweise repräsentiert die erste Repräsentation den Untersuchungsbereich ohne Kontrastmittel.

Die zweite Repräsentation repräsentiert den Untersuchungsbereich nach der Applikation einer zweiten Menge eines Kontrastmittels. Die zweite Menge ist größer als die erste Menge (wobei die erste Menge wie beschrieben auch Null sein kann). Der Ausdruck "nach der zweiten Menge eines Kontrastmittels" soll nicht so verstanden werden, dass sich die erste Menge und die zweite Menge in dem Untersuchungsbereich addieren (es sei denn die erste Menge ist Null). Der Ausdruck "die Repräsentation repräsentiert den Untersuchungsbereich nach der Applikation einer (ersten oder zweiten) Menge" soll also eher bedeuten: "die Repräsentation repräsentiert den Untersuchungsbereich mit einer (ersten oder zweiten) Menge" oder "die Repräsentation repräsentiert den Untersuchungsbereich umfassend eine (erste oder zweite) Menge".

Ist die erste Menge ungleich Null, dann repräsentieren die erste Repräsentation und die zweite Repräsentation den Untersuchungsbereich vorzugsweise (aber nicht notwendigerweise) in dem gleichen zeitlichen Abstand zur Applikation des Kontrastmittels. Ist die erste Menge gleich Null, kann der zeitliche Abstand zum Zeitpunkt der Applikation des Kontrastmittel bei der zweiten Repräsentation beliebig gewählt werden.

Vorzugsweise ist sowohl die erste Menge als auch die zweite Menge des Kontrastmittels kleiner als die Standardmenge.

Die Standardmenge ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

So beträgt die Standardmenge von Primovist^{®} beispielsweise 0,025 mmol Gd-EOB-DTPA Dinatrium / kg Körpergewicht.

In einer Ausführungsform der vorliegenden Offenbarung handelt es sich bei dem Kontrastmittel um ein Mittel, das Gadolinium(III) 2-[4,7,10-tris(carboxymethyl)-1,4,7,10-tetrazacyclododec-1-yl]essigsäure (auch als Gadolinium-DOTA oder Gadotersäure bezeichnet) umfasst.

In einer weiteren Ausführungsform der vorliegenden Offenbarung handelt es sich bei dem Kontrastmittel um ein Mittel, das Gadolinium(III) Ethoxybenzyl-diethylenetriaminepentaessigsäure (Gd-EOB-DTPA) umfasst; vorzugsweise umfasst das Kontrastmittel das Dinatriumsalz der Gadolinium(III)-ethoxybenzyl-diethylenetriaminpentaessigsäure (auch als Gadoxetsäure bezeichnet).

In einer Ausführungsform der vorliegenden Offenbarung handelt es sich bei dem Kontrastmittel um ein Mittel, das Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat (auch als Gadopiclenol bezeichnet, siehe z.B. WO2007/042504 sowie WO2020/030618 und/oder WO2022/013454) umfasst.

In einer Ausführungsform der vorliegenden Offenbarung handelt es sich bei dem Kontrastmittel um ein Mittel, das Dihydrogen[(±)-4-carboxy-5,8,11-tris(carboxymethyl)-1-phenyl-2-oxa-5,8,11-triazatridecan-13-oato(5-)]gadolinat(2-) (auch als Gadobensäure bezeichnet) umfasst.

In einer Ausführungsform der vorliegenden Offenbarung handelt es sich bei dem Kontrastmittel um ein Mittel, das Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat (auch als Gadoquatrane bezeichnet) umfasst (siehe z.B. J. Lohrke et al.: Preclinical Profile of Gadoquatrane: A Novel Tetrameric, Macrocyclic High Relaxivity Gadolinium-Based Contrast Agent. Invest Radiol., 2022, 1, 57(10): 629-638; WO2016193190).

In einer Ausführungsform der vorliegenden Offenbarung handelt es sich bei dem Kontrastmittel um ein Mittel, das einen Gd³⁺-Komplex einer Verbindung der Formel (I) 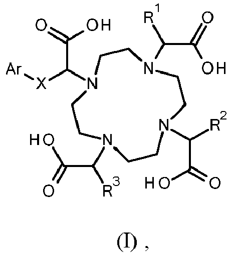 umfasst, wobei
Ar eine Gruppe ausgewählt aus 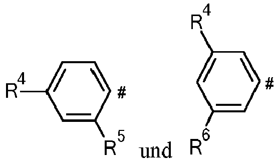 darstellt,
   wobei _{#} die Anknüpfung zu X darstellt,
X eine Gruppe darstellt, die aus
CH₂, (CH₂)₂, (CH₂)₃, (CH₂)₄ und ^{∗}-(CH₂)₂-O-CH₂-^{#} ausgewählt wird,
wobei ^{∗} die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
   und
R⁶ ein Wasserstoffatom darstellt,
   oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen Gd³⁺-Komplex einer Verbindung der Formel (II) 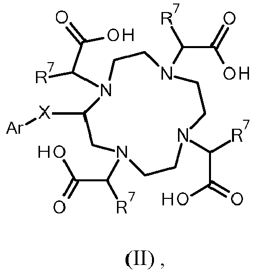 umfasst, wobei
Ar eine Gruppe ausgewählt aus 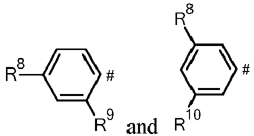 darstellt,
   wobei _{#} die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
   oder ein Stereoisomer, Tautomer, Hydrat, Solvat oder Salz davon, oder eine Mischung davon.

Der Begriff "C₁-C₃-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "C₂-C₄-Alkyl" bedeutet eine lineare oder verzweigte, gesättigte, einwertige Kohlenwasserstoffgruppe mit 2, 3 oder 4 Kohlenstoffatomen.

Der Begriff "C₂-C₄-Alkoxy" bedeutet eine lineare oder verzweigte, gesättigte, einwertige Gruppe der Formel (C₂-C₄-Alkyl)-O-, in der der Begriff "C₂-C₄-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2). In einer Ausführungsform der vorliegenden Offenbarung handelt es sich bei dem Kontrastmittel um ein Mittel, das Gadolinium 2,2',2"-{10-[(1R)-1-carboay-2- {4-[2-(2-ethoxyethoxy)ethoxy]phenyl}ethyl]-1,4,7,10-tetraazacyclododecan-1,4,7-triyl}triacetatumfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat (auch als Gadobutrol oder Gd-DO3A-butrol bezeichnet) umfasst.

In einem ersten Schritt werden die erste Repräsentation und die zweite Repräsentation empfangen oder erzeugt.

Der Begriff "Empfangen" umfasst sowohl das Abrufen von Repräsentationen als auch das Entgegennehmen von Repräsentationen, die z.B. an das Computersystem der vorliegenden Offenbarung übermittelt werden. Die Repräsentationen können von einem Computertomographen, von einem Magnetresonanztomographen oder von einem Ultraschall-Scanner empfangen werden. Die radiologischen Aufnahmen können aus einem oder mehreren Datenspeichern ausgelesen und/oder von einem separaten Computersystem übermittelt werden.

Üblicherweise fallen radiologische Aufnahmen als Ergebnis von radiologischen Untersuchungen (insbesondere im Fall der Computertomografie und der Sonografie) als Repräsentationen im Ortsraum (auch Bildraum genannt) an. Die erste Repräsentation und die zweite Repräsentation, die in einem ersten Schritt empfangen oder erzeugt werden, sind Repräsentationen im Ortsraum.

Der "Ortsraum" ist der gewöhnliche dreidimensionale Euklidische Raum, der mit dem Raum übereinstimmt, den wir Menschen mit unseren Sinnen erfahren und in dem wir uns fortbewegen. Eine Repräsentation im Ortsraum ist daher die für Menschen geläufige Repräsentation.

In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (Pixel oder Voxel) repräsentiert, die beispielsweise rasterförmig angeordnet sein können, wobei jedes Bildelement einen Teil des Untersuchungsbereichs repräsentiert, wobei jedem Bildelement ein Farbwert oder Grauwert zugeordnet sein kann. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement. Andere Formate zur Speicherung, Verarbeitung und/oder Darstellung von Repräsentationen im Ortsraum sind auch möglich.

Es ist möglich, eine Co-Registrierung von Repräsentationen im Ortsraum durchzuführen. Die "Co-Registrierung" (im Stand der Technik auch "Bildregistrierung" genannt) dient dazu, zwei oder mehrere Ortsraumdarstellungen desselben Untersuchungsbereichs bestmöglich in Übereinstimmung miteinander zu bringen. Dabei wird eine der Ortsraumdarstellungen als Referenzbild festgelegt, die andere wird Objektbild genannt. Um diese optimal an das Referenzbild anzupassen, wird eine ausgleichende Transformation berechnet.

Auf Basis der ersten Repräsentation und der zweiten Repräsentation wird eine dritte Repräsentation erzeugt.

Die dritte Repräsentation repräsentiert zumindest einen Teil des Untersuchungsbereichs des Untersuchungsobjekts ebenfalls im Ortsraum.

Die dritte Repräsentation repräsentiert die durch die zweite Menge an Kontrastmittel im Untersuchungsbereich hervorgerufene Signalverstärkung. Mit anderen Worten: die dritte Repräsentation umfasst die Unterschiede in der zweiten Repräsentation gegenüber der ersten Repräsentation, die durch die zweite Menge an Kontrastmittel hervorgerufen werden.

In einer bevorzugten Ausführungsform umfasst die Erzeugung der dritten Repräsentation ein Subtrahieren der ersten Repräsentation von der zweiten Repräsentation. Mit anderen Worten: in einer bevorzugten Ausführungsform ist die dritte Repräsentation die Differenz der ersten und der zweiten Repräsentation.

In einem weiteren Schritt wird eine vierte Repräsentation durch Kombinieren der ersten Repräsentation mit der dritten Repräsentation erzeugt. Durch eine solche Kombination werden Informationen zu der Kontrastverstärkung, die durch die zweite Menge des Kontrastmittels im Untersuchungsbereich hervorgerufen wird, auf die erste Repräsentation übertragen.

Die Kombination kann beispielsweise ein α-faches Addieren der dritten Repräsentation zu der ersten Repräsentation sein oder umfassen, wobei α eine positive oder negative reelle Zahl ist. Mit anderen Worten: die dritte Repräsentation wird mit einem Verstärkungsfaktor α multipliziert und das Ergebnis der Multiplikation zu der ersten Repräsentation addiert. Die mathematischen Methoden (Subtrahieren, Addieren, Multiplizieren) betreffen dabei üblicherweise die Tonwerte der einzelnen Bildelemente (Pixel, Voxel).

Der Verstärkungsfaktor kann von einem Nutzer gewählt werden, d.h. variabel sein, oder vordefiniert sein, d.h. vorgegeben sein.

Der Verstärkungsfaktor gibt an, in welchem Maß der Kontrast in der vierten Repräsentation erhöht wird.

Die vierte Repräsentation kann ausgegeben werden (z.B. auf einem Bildschirm angezeigt oder mit Hilfe eines Druckers ausgedruckt werden), auf einem Datenspeicher gespeichert werden und/oder an ein separates Computersystem übermittelt werden.

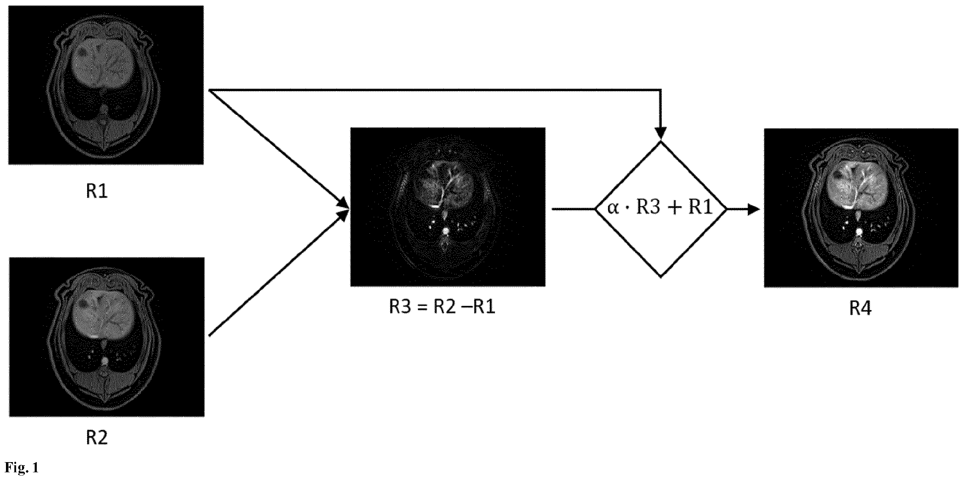

Fig. 1 zeigt die Erzeugung einer dritten Repräsentation und einer vierten Repräsentation auf Basis einer ersten Repräsentation und einer zweiten Repräsentation schematisch in Form eines Beispiels.

Fig. 1 zeigt einen Untersuchungsbereich eines Untersuchungsobjekts in Form von verschiedenen Repräsentationen.

Eine erste Repräsentation R1 repräsentiert den Untersuchungsbereich im Ortsraum ohne Kontrastmittel. Der in Fig. 1 gezeigte Untersuchungsbereich umfasst eine Leber eines Schweines. Die erste Repräsentation R1 ist eine magnetresonanztomografische Aufnahme.

Eine zweite Repräsentation R2 repräsentiert denselben Untersuchungsbereich desselben Untersuchungsobjekts wie die erste Repräsentation R1 im Ortsraum. Die zweite Repräsentation R2 ist ebenfalls eine magnetresonanztomografische Aufnahme.

Die zweite Repräsentation R2 repräsentiert den Untersuchungsbereich nach der Applikation einer Menge eines Kontrastmittels. Im vorliegenden Beispiel wurde dem Untersuchungsobjekt eine Menge von 25 µmol pro kg Körpergewicht eines hepatobiliären Kontrastmittels intravenös verabreicht. Die zweite Repräsentation R2 repräsentiert den Untersuchungsbereich in der so genannten arteriellen Phase.

Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist unter dem Markennamen Primovist^{®} und Eovist^{®} kommerziell erhältlich ist. Weitere hepatobiliäre Kontrastmittel sind u.a. in WO2022/194777 beschrieben.

Auf Basis der ersten Repräsentation R1 und der zweiten Repräsentation R2 wird eine dritte Repräsentation R3 erzeugt. In dem in Fig. 1 gezeigten Beispiel ist die dritte Repräsentation R3 die Differenz der zweiten Repräsentation R2 und der ersten Repräsentation R1 (R3=R2-R1).

In der dritten Repräsentation können negative Tonwerte, die bei der Subtraktion der ersten Repräsentation von der zweiten Repräsentation entstehen können, auf Null (oder einen anderen Wert) gesetzt werden, um negative Werte zu vermeiden.

Die dritte Repräsentation R3 repräsentiert die durch die zweite Menge des Kontrastmittels im Untersuchungsbereich hervorgerufene Kontrastverstärkung.

Die dritte Repräsentation R3 wird mit dem Verstärkungsfaktor α multipliziert und das Ergebnis der Multiplikation zu der ersten Repräsentation R1 addiert. Auf diese Weise wird eine vierte Repräsentation R4 erzeugt. In dem in Fig. 1 gezeigten Beispiel beträgt der Verstärkungsfaktor α=3, d.h. die dritte Repräsentation R3 wird dreimal zu der ersten Repräsentation R1 addiert.

Die vierte Repräsentation R4 kann einer Normalisierung unterzogen werden, das heißt, die Tonwerte können mit einem Faktor multipliziert werden, so dass der Tonwert mit dem höchsten Wert beispielsweise durch den Farbton weiß und der Tonwert mit dem geringsten Wert beispielsweise durch den Farbton schwarz repräsentiert wird.

Es auch möglich, einen Verstärkungsfaktor zu wählen, die kleiner als 1 ist, d.h. der Kontrast zwischen Bereichen mit Kontrastmittel und Bereichen ohne Kontrastmittel ist dann in der vierten Repräsentation geringer als in der zweiten Repräsentation.

Es ist auch möglich, den Kontrast durch negative Werte des Verstärkungsfaktors α weiter zu verringern. Dies kann zum Beispiel nützlich sein, um auf Basis einer ersten Repräsentation, die den Untersuchungsbereich mit einer ersten Menge eines Kontrastmittels repräsentiert, und einer zweiten Repräsentation, die den Untersuchungsbereich mit einer zweiten Menge des Kontrastmittels repräsentiert, eine Repräsentation des Untersuchungsbereichs ohne Kontrastmittel zu erzeugen. Ist die zweite Menge beispielsweise doppelt so groß wie die erste Menge, ergibt sich bei einem Verstärkungsfaktor von α =-1 eine vierte Repräsentation, die so aussieht, als ob kein Kontrastmittel appliziert worden wäre, vorausgesetzt, die durch das Kontrastmittel hervorgerufene Signalverstärkung steigt linear mit der Menge an und die erste und die zweite Repräsentation repräsentieren den Untersuchungsbereich in gleichem zeitlichen Abstand zu der Applikation der ersten bzw. zweiten Menge.

Es ist auch möglich negative Werte von α so zu wählen, dass Bereiche des Untersuchungsbereichs, die eine Kontrastmittel-induzierte Signal-Verstärkung in der messtechnisch erzeugten Repräsentation erfahren, in den künstlich erzeugten Repräsentationen komplett dunkel (schwarz) sind.

Ebenso ist es möglich, eine Verstärkung zu erzielen, die größer ist als die durch eine Standardmenge an Kontrastmittel hervorgerufene Kontrastverstärkung. Eine solche Kontrastverstärkung ist mit dem in WO2019/074938A1 beschriebenen Verfahren nicht möglich, ohne dass man zur Erzeugung der Trainingsdaten Personen eine Menge an Kontrastmittel appliziert, die höher als die Standardmenge ist und damit außerhalb des von der Zulassungsbehörde zugelassenen Bereichs liegt.

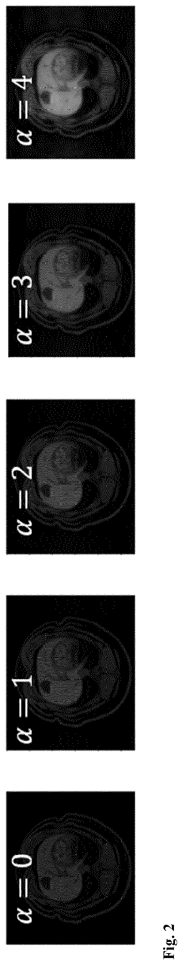

Fig. 2 zeigt beispielhaft und schematisch verschiedene Repräsentationen eines Untersuchungsbereichs eines Untersuchungsobjekts. Die Repräsentationen unterscheiden sich durch den Verstärkungsfaktor a, der in den vorliegenden Beispielen die Werte 0, 1, 2 ,3 und 4 annehmen kann.

Ein Verstärkungsfaktor von α = 0 bedeutet, dass bei der ersten Repräsentation keine Kontrastverstärkung vorgenommen wird.

Ein Verstärkungsfaktor von α = 1 bedeutet, dass zu der ersten Repräsentation die dritte Repräsentation einmal addiert wird. Die Kontrastverstärkung entspricht jener der zweiten Repräsentation.

Ein Verstärkungsfaktor von α = 2, 3 oder 4 bedeutet, dass zu der ersten Repräsentation die dritte Repräsentation 2-mal, 3-mal oder 4-mal addiert wird. Die Kontrastverstärkung steigt mit zunehmendem Verstärkungsfaktor an.

In dem in Fig. 2 gezeigten Beispiel wird stets ein ganzzahliges Vielfaches der dritten Repräsentation zu der ersten Repräsentation addiert. Natürlich ist es auch möglich, einen nichtganzzahligen Anteil der dritten Repräsentation zu der ersten Repräsentation zu addieren (z.B. α = 1,5 oder α = 3,7 oder α = 4,159). Somit kann eine Verstärkung kontinuierlich gesteigert werden.

Es ist auch möglich, den Verstärkungsfaktor automatisch zu ermitteln. Es ist z.B. möglich, in einer Darstellung der ersten und/oder der zweiten Repräsentation (zum Beispiel auf einem Bildschirm) mindestens einen Bereich zu definieren und/oder von einem Nutzer auswählen zu lassen und den Verstärkungsfaktor so einzustellen, dass ein Grauwert in der Darstellung (oder ein anderer Tonwert im Falle einer anderen Darstellung als einer Grauwertdarstellung) einen definierten Wert annimmt und/oder oberhalb oder unterhalb eines Schwellenwerts liegt und/oder zwei Grauwerte in zwei verschiedenen ausgewählten oder definierten Bereichen einen definierten Abstand voneinander haben und/oder einen Abstand voneinander aufweisen, der oberhalb oder unterhalb eines Schwellenwerts liegt. Es ist auch möglich, andere Kriterien anzuwenden, um den Verstärkungsfaktor automatisch zu ermitteln. Grundlage für die Kriterien zur automatischen Ermittlung des Verstärkungsfaktor kann beispielsweise das Histogramm der ersten, zweiten, dritten und/oder vierten Repräsentation sein. In einem solchen Histogramm können die Zahl der Bildelemente mit einem definierten Tonwert oder Grauwert aufgeführt sein.

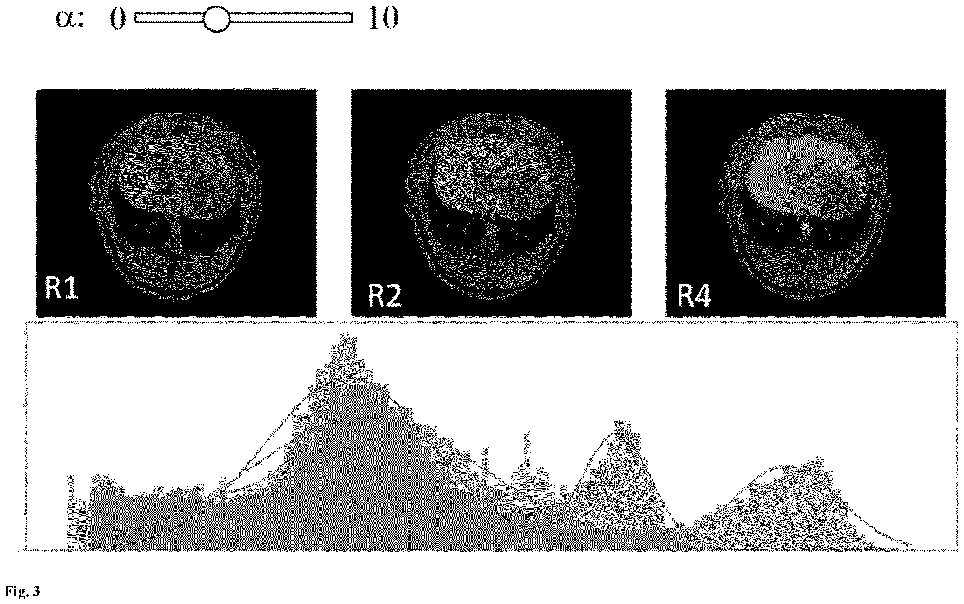

Fig. 3 zeigt eine bevorzugte Ausführungsform einer Ausgabe der künstlichen kontrastverstärkten radiologischen Aufnahme eines Untersuchungsbereichs mittels eines Computersystems/Computerprogramms. Die Ausgabe erfolgt gegenüber einem Nutzer des Computersystems und/oder Computerprogramms der vorliegenden Offenbarung.

Dem Nutzer werden eine erste Repräsentation R1 eines Untersuchungsbereichs eines Untersuchungsobjekts, eine zweite Repräsentation R2 des Untersuchungsbereichs des Untersuchungsobjekts und eine vierte Repräsentation R4 des Untersuchungsbereichs des Untersuchungsobjekts angezeigt (zum Beispiel auf einem Monitor).

Die erste Repräsentation R1 repräsentiert den Untersuchungsbereich ohne Kontrastmittel oder nach der Applikation einer ersten Menge eines Kontrastmittels.

Die zweite Repräsentation R2 repräsentiert den Untersuchungsbereich nach der Applikation einer zweiten Menge des Kontrastmittels. Die zweite Menge ist größer als die erste Menge.

Die vierte Repräsentation R4 repräsentiert den Untersuchungsbereich mit einem verstärkten Kontrast. Der Kontrast zwischen Bereichen ohne Kontrastmittel und Bereichen mit Kontrastmittel ist im Fall der vierten Repräsentation R4 größer als bei der zweiten Repräsentation R2.

Die vierte Repräsentation R4 wurde wie in dieser Offenbarung beschrieben (siehe insbesondere die Beschreibungen zu Fig. 1) erzeugt.

Unterhalb der angezeigten Repräsentationen R1, R2 und R4 werden dem Nutzer die Histogramme der Repräsentationen in einer überlagerten Darstellung angezeigt.

Oberhalb der angezeigten Repräsentationen R1, R2 und R4 wird dem Nutzer ein virtueller Schieberegler zur Verfügung gestellt, mit dem der Nutzer Einstellungen vornehmen kann. Mit diesem Schieberegler kann der Nutzer den Verstärkungsfaktor α im Bereich von 1 bis 10 beliebig wählen. Der Schieberegler zeigt an, dass eine kontinuierliche Anhebung des Verstärkungsfaktor von 1 auf 10 möglich ist.

Die in Fig. 3 gezeigte Ausgabe ist vorzugsweise so konfiguriert, dass die Anzeige der vierten Repräsentation R4 unmittelbar aktualisiert wird, wenn der Nutzer Änderungen mit dem Schieberegler vornimmt. Der Nutzer kann dann beispielsweise den Verstärkungsfaktor α ändern und unmittelbar sehen, wie sich eine Änderung des Verstärkungsfaktors auf die Repräsentation R4 auswirkt. So kann er diejenigen Einstellungen identifizieren, die zu einer für den Nutzer optimalen vierten Repräsentation R4 des Untersuchungsbereichs führen.

Jede Änderung des Parameters α führt bei dem Computersystem dazu, dass auf Basis des geänderten Parameters die vierte Repräsentation R4 jeweils neu im Hintergrund berechnet und angezeigt wird. Dies gilt analog auch für das Histogramm der vierten Repräsentation R4.

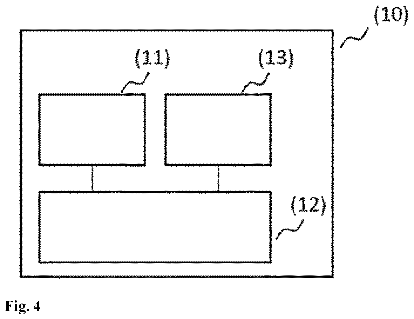

Fig. 4 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 4 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert:
- die Empfangseinheit zu veranlassen, eine erste Repräsentation zu empfangen, wobei die erste Repräsentation einen Untersuchungsbereich eines Untersuchungsobjekts ohne Kontrastmittel oder nach der Applikation einer ersten Menge eines Kontrastmittels repräsentiert,
- die Empfangseinheit zu veranlassen, eine zweite Repräsentation zu empfangen, wobei die zweite Repräsentation den Untersuchungsbereich des Untersuchungsobjekts nach einer Applikation einer zweiten Menge des Kontrastmittels repräsentiert,
- eine dritte Repräsentation auf Basis der ersten Repräsentation und der zweiten Repräsentation zu erzeugen, wobei das Erzeugen der dritten Repräsentation ein Subtrahieren der ersten Repräsentation von der zweiten Repräsentation umfasst,
- eine vierte Repräsentation auf Basis der ersten Repräsentation und der dritten Repräsentation zu erzeugen, wobei das Erzeugen der vierten Repräsentation ein α-faches Addieren der dritten Repräsentation zu der ersten Repräsentation umfasst, wobei α eine positive oder negative reelle Zahl ist,
- die Ausgabeeinheit zu veranlassen, die vierte Repräsentation auszugeben und/oder zu speichern und/oder an ein separates Computersystem zu übermitteln.

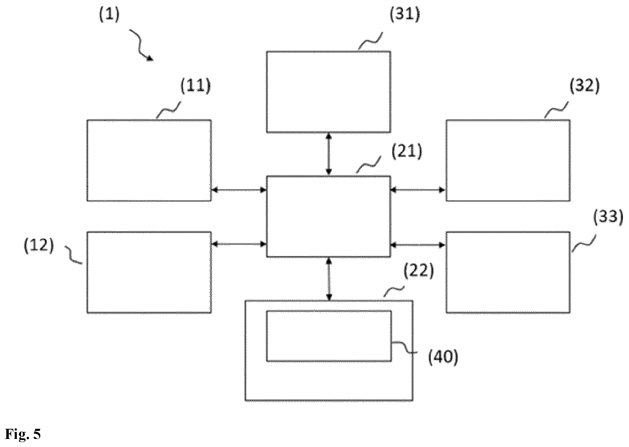

Fig. 5 zeigt beispielhaft und schematisch eine weitere Ausführungsform des Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 4 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

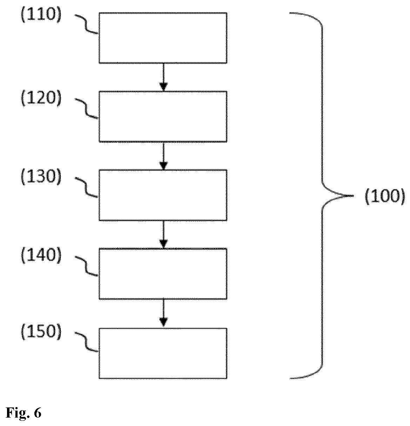

Fig. 6 zeigt beispielhaft und schematisch eine Ausführungsform des computer-implementierten Verfahrens in Form eines Ablaufschemas.

Das Verfahren (100) umfasst die Schritte:
- (110): Empfangen oder Erzeugen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- (120): Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- (130): Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- (140): Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- (150): Ausgeben und/oder Speichern der vierten Repräsentation und/oder Übermitteln der vierten Repräsentation des Untersuchungsbereichs an ein separates Computersystem.

## Patentansprüche

1. Computer-implementiertes Verfahren umfassend:
- Empfangen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

2. Verfahren nach Anspruch 1, wobei das Untersuchungsobjekt ein Lebewesen, vorzugsweise ein Säugetier, ganz besonders bevorzugt ein Mensch ist.

3. Verfahren nach Anspruch 1 oder 2, wobei der Untersuchungsbereich eine Leber, eine Niere, ein Herz, eine Lunge, ein Gehirn, ein Magen, eine Blase, eine Prostatadrüse, ein Darm und/oder ein Teil davon und/oder einen anderen/weiteren Teil des Körpers eines Menschen umfasst.

4. Verfahren nach einem der Ansprüche 1 bis 3, wobei α größer als 1 ist.

5. Verfahren nach einem der Ansprüche 1 bis 4, ferner umfassend:
- Empfangen eines oder mehrerer Werte für α von einem Nutzer.

6. Verfahren nach einem der Ansprüche 1 bis 5, ferner umfassend:
- Empfangen eines ersten Tonwerts eines ersten Bildelements einer Darstellung der ersten Repräsentation oder der zweiten Repräsentation,
- Empfangen eines zweiten Tonwerts eines zweiten Bildelements einer Darstellung der Repräsentation oder der zweiten Repräsentation,
- Ermitteln eines Wertes für a, für den die Differenz zwischen dem ersten Tonwert und dem zweiten Tonwert einen vordefinierten Wert annimmt oder oberhalb oder unterhalb eines vordefinierten Schwellenwerts liegt.

7. Verfahren nach einem der Ansprüche 1 bis 6, wobei die erste Repräsentation und die zweite Repräsentation das Ergebnis einer magnetresonanztomographischen Untersuchung sind und/oder aus Magnetresonanzaufnahmen erzeugt worden sind.

8. Verfahren nach einem der Ansprüche 1 bis 7, wobei das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)] gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2'-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl] acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(25)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

9. Computersystem umfassend
einen Prozessor; und
einen Speicher, der ein Anwendungsprogramm speichert, das so konfiguriert ist, dass es, wenn es vom Prozessor ausgeführt wird, eine Operation durchführt, wobei die Operation umfasst:
- Empfangen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

10. Computerprogrammprodukt umfassend einen Datenträger, auf dem ein Computerprogramm gespeichert ist, wobei das Computerprogramm in einen Arbeitsspeicher eines Computersystems geladen werden kann und dort das Computersystem dazu veranlasst, folgende Schritte ausführen:
- Empfangen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Empfangen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

11. Verwendung eines Kontrastmittels in einem radiologischen Untersuchungsverfahren umfassend:
- Empfangen oder Erzeugen einer ersten Repräsentation, wobei die erste Repräsentation einen Untersuchungsbereich eines Untersuchungsobjekts ohne das Kontrastmittel oder nach Applikation einer ersten Menge des Kontrastmittels repräsentiert,
- Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

12. Verwendung gemäß Anspruch 11, wobei das radiologische Untersuchungsverfahren eine magnetresonanztomographische Untersuchung ist und wobei das Kontrastmittel einen Gd³⁺-Komplex einer Verbindung der Formel (I) 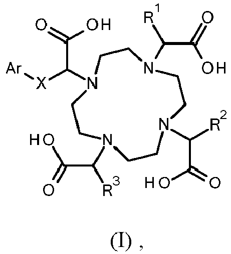 umfasst, wobei
Ar eine Gruppe ausgewählt aus 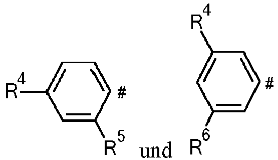 darstellt,
wobei _{#} die Anknüpfung zu X darstellt,
X eine Gruppe darstellt, die aus
CH₂, (CH₂)₂, (CH₂)₃, (CH₂)₄ und ^{∗}-(CH₂)₂-O-CH₂-^{#} ausgewählt wird,
wobei ^{∗} die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon.

13. Verwendung gemäß Anspruch 11, wobei das radiologische Untersuchungsverfahren eine magnetresonanztomographische Untersuchung ist und wobei das Kontrastmittel einen Gd³⁺-Komplex einer Verbindung der Formel (II) 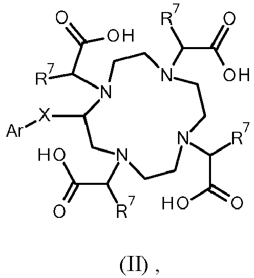 umfasst, wobei
Ar eine Gruppe ausgewählt aus 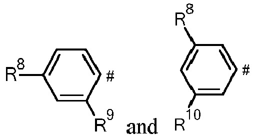 darstellt,
wobei _{#} die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.

14. Verwendung gemäß Anspruch 11, wobei das radiologische Untersuchungsverfahren eine magnetresonanztomographische Untersuchung ist und wobei das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5 -oxopentanoat,
- Dihydrogcn[(±)-4-carboxy-5,8,11-tris(carboxymethyl)-1-phenyl-2-oxa-5,8,11-triazatridecan-13-oato(5-)] gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-\yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoayphenyl)-1-carboaybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(25)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

15. Kontrastmittel zur Verwendung in einem radiologischen Untersuchungsverfahren umfassend:
- Empfangen oder Erzeugen einer ersten Repräsentation, wobei die erste Repräsentation einen Untersuchungsbereich eines Untersuchungsobjekts ohne das Kontrastmittel oder nach Applikation einer ersten Menge des Kontrastmittels repräsentiert,
- Empfangen oder Erzeugen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Erzeugen einer dritten Repräsentation auf Basis der ersten Repräsentation und der zweiten Repräsentation, wobei das Erzeugen der dritten Repräsentation ein Subtrahieren der ersten Repräsentation von der zweiten Repräsentation umfasst,
- Erzeugen einer vierten Repräsentation, wobei das Erzeugen der vierten Repräsentation ein α-faches Addieren der dritten Repräsentation zu der ersten Repräsentation umfasst, wobei α eine positive oder negative reelle Zahl ist,
- Ausgeben und/oder Speichern der vierten Repräsentation und/oder Übermitteln der vierten Repräsentation an ein separates Computersystem.

16. Kontrastmittel zur Verwendung gemäß Anspruch 15, wobei das radiologische Untersuchungsverfahren eine magnetresonanztomographische Untersuchung ist und wobei das Kontrastmittel einen Gd³⁺-Komplex einer Verbindung der Formel (I) 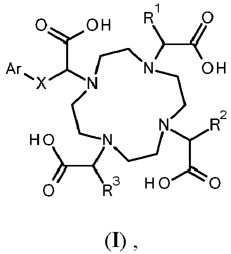 umfasst, wobei
Ar eine Gruppe ausgewählt aus 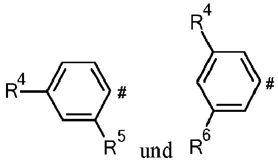 darstellt,
wobei _{#} die Anknüpfung zu X darstellt,
X eine Gruppe darstellt, die aus
CH₂, (CH₂)₂, (CH₂)₃, (CH₂)₄ und ^{∗}-(CH₂)₂-O-CH₂-^{#} ausgewählt wird,
wobei ^{∗} die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon.

17. Kontrastmittel zur Verwendung gemäß Anspruch 15, wobei das radiologische Untersuchungsverfahren eine magnetresonanztomographische Untersuchung ist und wobei das Kontrastmittel einen Gd³⁺-Komplex einer Verbindung der Formel (II) 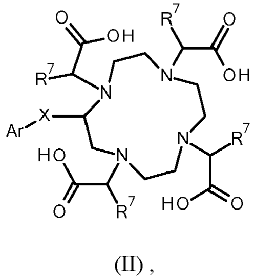 umfasst, wobei
Ar eine Gruppe ausgewählt aus 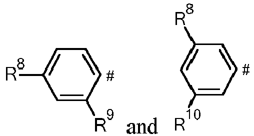 darstellt,
wobei _{#} die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.

18. Kontrastmittel zur Verwendung gemäß Anspruch 15, wobei das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5 -oxopentanoat,
- Dihydrogen[(±)-4-carboxy-5,8,11-tris(carboxymethyl)-1-phenyl-2-oxa-5,8,11-triazatridecan-13-oato(5-)] gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino }methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(25)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

19. Kit umfassend ein Computerprogrammprodukt gemäß Anspruch 10 und ein Kontrastmittel.

20. Kit gemäß Anspruch 19, wobei das Kontrastmittel einen Gd³⁺-Komplex einer Verbindung der Formel (I) 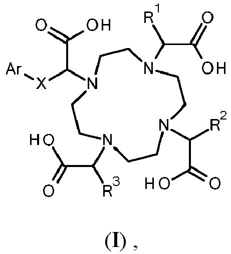 umfasst, wobei
Ar eine Gruppe ausgewählt aus 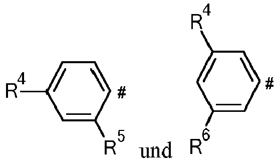 darstellt,
wobei _{#} die Anknüpfung zu X darstellt,
X eine Gruppe darstellt, die aus
CH₂, (CH₂)₂, (CH₂)₃, (CH₂)₄ und ^{∗}-(CH₂)₂-O-CH₂-^{#} ausgewählt wird,
wobei ^{∗} die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon.

21. Kit gemäß Anspruch 19, wobei das Kontrastmittel einen Gd³⁺-Komplex einer Verbindung der Formel (II) 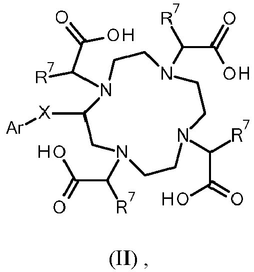 umfasst, wobei
Ar eine Gruppe ausgewählt aus 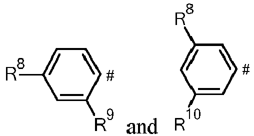 darstellt,
wobei _{#} die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.

22. Kit gemäß Anspruch 19, wobei das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5 -oxopentanoat,
- Dihydrogen[(±)-4-carboxy-5,8,11-tris(carboxymemyl)-1-phenyl-2-oxa-5,8,11-triazatridecan-13-oato(5-)] gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1yl]propanoyl}amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(25)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.
